# FASCICULE DE BREVET EUROPEEN

(11) **EP 0 966 276 B1**
(45) Date de publication et mention de la délivrance du brevet: **10.12.2003**
(21) Numéro de dépôt: 98903099.4
(22) Date de dépôt: 21.01.1998
(51) Int. Cl.: A61K 31/136, A61K 31/137, A61K 31/138, A61P 17/02

(54) **UTILISATION D'AGONISTES DES RECEPTEURS BETA-3 ADRENERGIQUES POUR LA PREPARATION DE MEDICAMENTS CICATRISANTS**
VERWENDUNG VON BETA-3-ADRENOREZEPTORAGONISTEN ZUR HERSTELLUNG VON WUNDHEILMITTELN
USE OF BETA-3 ADRENERGIC RECEPTOR AGONISTS FOR PREPARING WOUND-HEALING PHARMACEUTICALS

(30) Priorité: 21.01.1997 FR 9700584
(43) Date de publication de la demande: 29.12.1999
(73) Titulaire: SANOFI-SYNTHELABO, 75013 Paris (FR)
(72) Inventeur: BERNAT, André, F-31270 Cugnaux (FR); HERBERT, Jean-Marc, F-31170 Tournefeuille (FR); ARNONE, Michèle, F-31520 Ramonville St. Agne (FR)
(74) Mandataire: Gillard, Marie-Louise
(86) Numéro de dépôt international: FR9800105
(87) Numéro de publication internationale: WO98031357

(56) Documents cités:
- WO-A-94/24090
- US-A- 5 502 078
- US-A- 5 597 843
- KURATONI: "Enhancement of gastric mucosal blood flow by beta-3 adrenergic agonists prevents indomethacin-induced anthral ulcer in the rat" J. PHARMACOL. EXP. THER., vol. 270, no. 2, 1994, pages 559-565, XP002044584
- SHEN: "Peripheral vascular effects of beta-3 adrenergic receptor stimulation in concious dogs" J. PHARMACOL. EXP. THER., vol. 268, no. 1, 1994, pages 466-473, XP002044585
- DATABASE WPI Week 9543 Derwent Publications Ltd., London, GB; AN 95-332486 XP002044587 & JP 07 228 543 A (FUJISAWA PHARM CO LTD)
- ARCH: "Prospects for beta-3-adrenoceptor agonists in the treatment of obesity and diabetes" INT. J. OBESITY, vol. 20, no. 3, 1996, pages 191-199, XP002044586

## Description

La présente invention concerne une nouvelle indication des agonistes des récepteurs bêta-3 adrénergiques.

Plus particulièrement, l'invention se rapporte à l'utilisation des agonistes des recepteurs bêta-3 adrénergiques pour la préparation de médicaments cicatrisants.

Il est connu que les agonistes des récepteurs bêta-3 adrénergiques sont susceptibles d'être utilisés pour le traitement de l'obésité et du diabète, bien que la preuve clinique de cette activité n'ait pas été apportée avec certitude.

Il est également connu que les agonistes des récepteurs bêta-3 adrénergiques, ci-après désignés brièvement "β₃-agonistes", ont été proposés comme spasmolytiques intestinaux, pour le traitement des maladies gastrointestinales, plus particulièrement de la maladie inflammatoire de l'intestin, (IBD de l'anglais "inflammatory bowel disease"), du syndrôme du colon irritable et pour la protection du tractus gastro-intestinal à l'égard des effets secondaires des médicaments anti-inflammatoires non-stéroïdiens.

Le terme "β₃-agonistes" inclut les composés qui sont des agonistes des récepteurs bêta qui ont été définis "atypiques" ou "non β₁ non β₂" et qui, maintenant, sont reconnus comme sous-type de récepteur adrénergique appelé "β₃".

Le traitement des blessures qui ne se cicatrisent pas représente un problème clinique sérieux, difficile à résoudre car la guérison des blessures implique une série complexe de phénomènes qui se superposent et qui sont difficiles à maîtriser dans leur globalité.

Des facteurs de croissance, notamment le facteur de croissance des fibroblastes basique (bFGF, de l'anglais "basic Fibroblast Growth Factor", Biol. Pharm. Bull., 1996, 19(4), 530-535) et le facteur des fibroblases acide (aFGF, de l'anglais "acidic Fibroblast Growth Factor", J. Invest. Dermatol., 1995, 104, 850-855) ainsi que la sphingosylphosphorylcholine, J. Invest. Dermatol., 1996, 106, 232-237, ont été proposés comme agents pour la cicatrisation des blessures.

Il a été maintenant trouvé que les β₃-agonistes agissent en accélerant la guérison des blessures dermiques, chez les mammifères diabétiques.

Ainsi, selon un de ses aspects, la présente invention se rapporte à l'utilisation des β₃-agonistes pour la préparation de médicaments cicatrisants.

Des β₃-agonistes utiles pour l'utilisation selon la présente invention sont représentés par la formule (I) 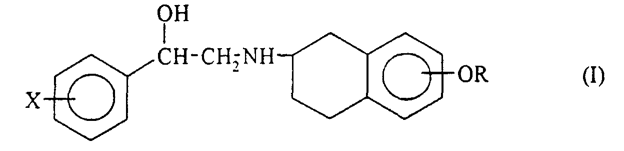 dans laquelle
- X: représente l'hydrogène, un halogène, un groupe trifluorométhyle ou un groupe (C₁-C₄)alkyle;
- R: représente l'hydrogène, un groupe méthyle, non substitué ou substitué par un groupe carboxy ou carbalcoxy inférieur
et leurs sels pharmaceutiquement acceptables, indiqués dans EP 0 211 721 et EP 0 303 546 qui décrivent les composés de formule (I) utiles comme spasmolytiques intestinaux.

Parmi les composés de formule (I), les composés:
- 2-[(7-hydroxy-1,2,3,4-tetrahydronapht-2-yl)amino]-1-phényléthanol;
- 2-[(7-hydroxy-1,2,3,4-tetrahydronapht-2-yl)amino]-1-(3-chlorophényl) éthanol;
- 2-[(7-carbéthoxyméthoxy-1,2,3,4-tetrahydronapht-2-yl)amino]-1-(3-chlorophényl)éthanol;
- 2-[(7-carbéthoxyméthoxy-1,2,3,4-tetrahydronapht-2-yl)amino]-1-phényléthanol;
- (1R,2'RS)-2-[(7-hydroxy-1,2,3,4-tetrahydronapht-2-yl)amino]-1-phényléthanol;
- (1S,2'RS)-2-[(7-hydroxy-1,2,3,4-tetrahydronapht-2-yl)amino]-1-phényléthanol;
- (+)-(1R)-2-[(7-hydroxy-1,2,3,4-tetrahydronapht-2-yl)amino]-1-phényléthanol;
- (+)-(1S)-2-[(7-hydroxy-1,2,3,4-tetrahydronapht-2-yl)amino]-1-phényléthanol;
- (-)-(1R)-2-[(7-hydroxy-1,2,3,4-tetrahydronapht-2-yl)amino]-1-phényléthanol;
- (-)-(1S)-2-[(7-hydroxy-1,2,3,4-tetrahydronapht-2-yl)amino]-1-phényléthanol;
- N-[(2R)-7-éthoxycarbonylméthoxy-1,2,3,4-tetrahydronapht-2-yl]-(2R)-2-(3-chlorophényl)-2-hydroxyéthanamine;
- N-[(2R)-7-méthoxycarbonylméthoxy-1,2,3,4-tetrahydronapht-2-yl]-(2R)-2-(3-chlorophényl)-2-hydroxyéthanamine;
- et, notamment, la N-[(2S)-7-éthoxycarbonylméthoxy-1,2,3,4-tetrahydronapht-2-yl]-(2R)-2-(3-chlorophényl)-2-hydroxyéthanamine (SR58611);
ainsi que leurs sels pharmaceutiquement acceptables, sont des composés particulièrement avantageux.

D'autres β₃-agonistes utiles pour l'utilisation selon la présente invention sont représentés par la formule (II) 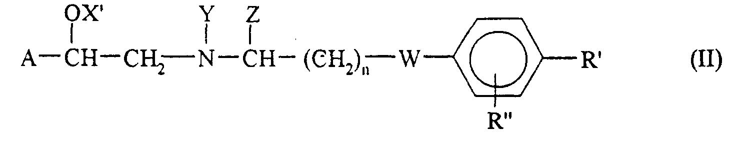 dans laquelle
- n: est 1, 2 ou 3;
- A: représente un groupe benzofuran-2-yle ou un groupe phényle non substitué ou substitué par un ou deux atomes d'halogène ou par un groupe (C₁-C₄)alkyle ou trifluorométhyle;
- R': représente
- un hydrogène;
- un groupe (C₁-C₆)alkyle;
- un groupe fonctionnel choisi parmi les groupements suivants: hydroxy; (C₁-C₆)alcoxy; (C₂-C₆)alcényloxy; (C₂-C₆)alcynyloxy; (C₃-C₈)cycloalkyloxy; (C₃-C₈)cycloalkyl(C₁-C₆)alcoxy; benzyloxy; phénoxy; mercapto; (C₁-C₆)alkylthio; (C₂-C₆)alcénylthio; (C₂-C₆)alcynylthio; (C₃-C₈)cycloalkylthio; (C₃-C₈)cycloalkyl(C₁-C₆)alkylthio; benzylthio; phénylthio; ((C₁-C₆)alkyl)sulfinyle; ((C₂-C₆)alcényl)sulfinyle; ((C₂-C₆)alcynyl)sulfinyle; (C₃-C₈)cycloalkylsulfinyle; ((C₃-C₈)cycloalkyl(C₁-C₆)alkyl)sulfinyle; benzylsulfinyle; phénylsulfinyle; ((C₁-C₆)alkyl) sulfonyle; ((C₂-C₆)alcényl)sulfonyle; ((C₂-C₆)alcynyl)sulfonyle; (C₃-C₈)cycloalkylsulfonyle; ((C₃-C₈)cycloalkyl(C₁-C₆)alkyl)sulfonyle; benzylsulfonyle; phénylsulfonyle; cyano; nitro; amino non substitué ou substitué par un ou deux radicaux, identiques ou différents choisis parmi (C₁-C₆)alkyle, (C₂-C₆)alcényle, (C₂-C₆)alcynyle, (C₃-C₈)cycloalkyle, (C₃-C₈)cycloalkyl(C₁-C₆)alkyle, benzyle, phényle; carboxy; carbalcoxy dont le groupe alkyle est en C₁-C₆; ((C₂-C₆)alcényloxy)carbonyle; ((C₂-C₆)alcynyloxy)carbonyle; (C₃-C₈)cycloalkyloxycarbonyle; ((C₃-C₈)cycloalkyl(C₁-C₆)alcoxy)carbonyle; benzyloxycarbonyle; phénoxycarbonyle; carbamoyle non substitué ou substitué sur le groupe amino par un ou deux radicaux, identiques ou différents, choisis parmi les groupes (C₁-C₆)alkyle, (C₂-C₆)alcényle, (C₂-C₆)alcynyle, (C₃-C₈)cycloalkyle, (C₃-C₈)cycloalkyl(C₁-C₆)alkyle, benzyle et phényle;
- un groupe R''' choisi parmi les groupes (C₁-C₆)alkyle substitué par un groupe fonctionnel; (C₂-C₆)alcényle substitué par un groupe fonctionnel; (C₂-C₆)alcynyle substitué par un groupe fonctionnel; phényl (C₁-C₆)alkyle substitué sur le groupe phényle par un (C₁-C₆)alkyle ou par un groupe fonctionnel; phényl(C₂-C₆)alcényle substitué sur le groupe phényle par un (C₁-C₆)alkyle ou par un groupe fonctionnel; phényl(C₂-C₆)alcynyle substitué sur le groupe phényle par un (C₁-C₆)alkyle ou par un groupe fonctionnel; benzyle substitué sur le groupe phényle par un (C₁-C₆)alkyle ou par un groupe fonctionnel; phényle, non substitué ou substitué par un (C₁-C₆)alkyle ou par un groupe fonctionnel; le groupe fonctionnel étant tel que défini ci-dessus;
- un groupe O-R''', S-R''', SO-R''', SO₂-R''' dans lesquels R''' est tel que défini ci-dessus pour R';
- un groupe NR'''R°, où R''' est tel que défini ci-dessus et R° représente l'hydrogène ou est tel que défini ci-dessus pour R''', ou bien R''' et R° forment, avec l'azote auquel ils sont liés, un groupe choisi parmi les groupes pyrrolidino, pipéridino et morpholino;
- un groupe COOR''' ou un groupe CO-SR''' dans lesquels R''' est tel que défini ci-dessus;
- un groupe CONR'''R° où R''' est tel que défini ci-dessus et R° représente l'hydrogène ou est tel que défini ci-dessus pour R"', ou bien R''' et R° forment, avec l'azote auquel ils sont liés, un groupe choisi parmi les groupes pyrrolidino, pipéridino et morpholino;
- un groupe SO₂NR'''R° où R''' est tel que défini ci-dessus et R° représente l'hydrogène ou est tel que défini ci-dessus pour R''', ou bien R''' et R° forment, avec l'azote auquel ils sont liés, un groupe choisi parmi les groupes pyrrolidino, pipéridino et morpholino;
- R": représente
- un hydrogène;
- un halogène;
- un groupe (C₁-C₆)alkyle;
- un groupe fonctionnel tel que défini ci-dessus pour R';
- un groupe OR''', R''' étant tel que défini ci-dessus;
- un groupe COOR''', R''' étant tel que défini ci-dessus;
- un groupe CONR'''R° où R''' est tel que défini ci-dessus et R° représente l'hydrogène ou est tel que défini ci-dessus pour R''', ou bien R''' et R° forment, avec l'azote auquel ils sont liés, un groupe choisi parmi les groupes pyrrolidino, pipéridino et morpholino;
- W: représente une liaison directe ou un atome d'oxygène;
- X': représente l'hydrogène, un (C₁-C₆)alkyle ou un (C₁-C₆)alkylcarbonyle;
- Y: représente l'hydrogène ou un groupe A'-CH(OH)-CH₂-, A' étant identique à A, mais autre que benzofuran-2-yle; ou bien
- X' et Y,: pris ensemble, forment un groupe méthylène, éventuellement substitué par un groupe carbalcoxy dont le groupe alkyle est en C₁-C₆; un groupe éthylène, éventuellement substitué par un groupe oxo; ou un groupe 1,3-propylène;
- Z: représente l'hydrogène ou un (C₁-C₆)alkyle;
ainsi que leurs sels pharmaceutiquement acceptables, indiqués dans EP 0 255 415 qui décrit l'utilisation des composés de formule (II) comme spasmolytiques intestinaux.

D'autres β₃-agonistes encore utiles pour l'utilisation selon la présente invention sont représentés par la formule (III) 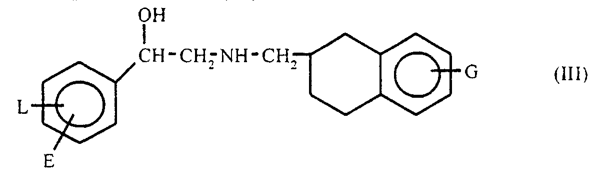 dans laquelle
- E: représente l'hydrogène, un groupe (C₁-C₄)alkyle, un groupe (C₁-C₄)alcoxy, un groupe phényle, un groupe nitro, un atome d'halogène ou un groupe trifluorométhyle,
- L: représente l'hydrogène, un groupe (C₁-C₄)alkyle, un groupe (C₁-C₄)alcoxy, un groupe phényle, un groupe nitro, un atome d'halogène ou
- E et L,: ensemble, représentent un groupe -CH=CH-CH=CH- ou -CH₂-CH₂-CH₂-CH₂-, et
- G: représente l'hydrogène, un atome de chlore, un groupe hydroxy ou un groupe OG' où G' représente un groupe (C₁-C₄)alkyle non substitué ou substitué par un groupe hydroxy, (C₁-C₄)alcoxy, (C₁-C₄)alcoxycarbonyle, carboxy ou (C₃-C₇)cycloalkyle; un groupe (C₃-C₇)cycloalkyle; un groupe (C₂-C₄)alcanoyle,
ainsi que leurs sels pharmaceutiquement acceptables, indiqués dans EP 0 436 435 qui décrit les composés de formule (III) utiles comme spasmolytiques intestinaux.

Parmi les composés de formule (III), la N-[(6-hydroxy-1,2,3,4-tétrahydronaphtalen-(2R)-2-yl)méthyl]-(2R)-2-hydroxy-2-(3-chlorophényl)éthanamine (SR 59104), la N-[(7-méthoxy-1,2,3,4-tétrahydronaphtalen-(2R)-2-yl)méthyl]-(2R)-2-hydroxy-2-(3-chlorophényl)éthanamine (SR 59119), ainsi que leurs sels pharmaceutiquement acceptables, sont des composés particulièrement avantageux.

D'autres composés β₃-agonistes avantageux selon la présente invention sont :
- le composé BRL 35135 décrit dans EP 23385, de formule : 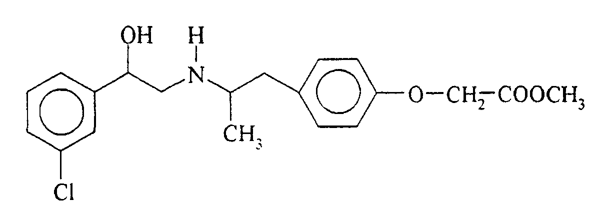
- le composé CL 316243 décrit dans US 5,061,727, de formule : 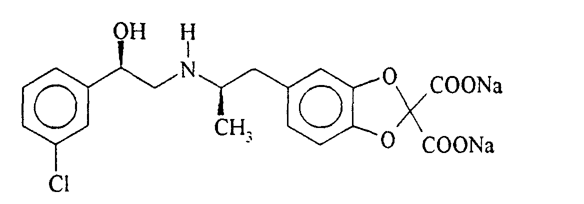
- le composé AZ 002 décrit dans EP 218440, de formule : 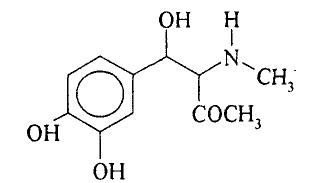
- le composé BMS 187257 décrit dans US 5,321,036, de formule : 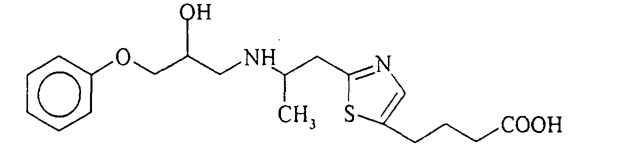
- le composé L-755507, de formule 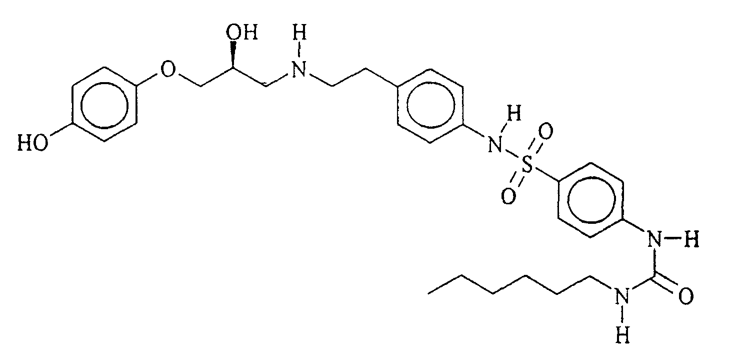 et le composé L-750355, de formule 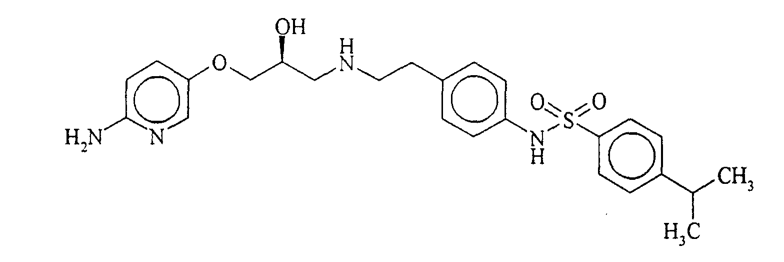 décrits dans EP611003 ;
- le composé FR-149175 décrit dans Japan J. Pharmacol., 1997, 74,(1):109, de formule : 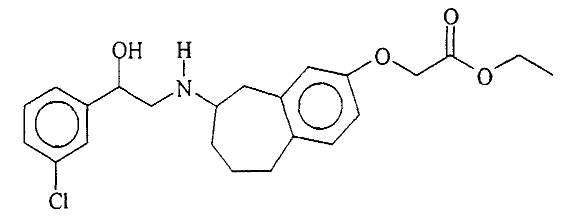
- le composé SB-226552, décrit dans Int. J. Obesity, 1997, 21, Suppl. 2 : 560 de formule : 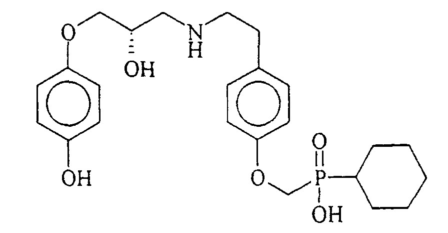 ainsi que les produits décrits dans les brevets/demandes de brevet suivants : WO 96/35671, WO 96/35670, WO 96/16038, WO 96/04233, WO 95/33724, WO 95/29159, EP 659737, WO 95/04047, EP 516349, EP 473285, EP 23385, EP 21636, EP 7205, JP 08198866, JP 08165276, JP 08157470, WO 96/16938, EP 714883, WO 96/04234, US 5,488,064, US 5,482,971, US 5,491,134, WO 95/29159, WO 95/33724, ZA 9409874, WO 95/29903, US 5,461,163, WO 95/25104, EP 659737, JP 07112958, WO 95/8527, WO 95/07284, JP 07025756, WO 95/03289, WO 95/04047, WO 95/01170, WO 94/29290, US 5,373,020, JP 06293664, WO 94/12166, EP 611003, WO 97/10825, WO 97/21666, WO 97/21665, JP 09118655, WO 97/15549, GB 2305665, EP 764640, EP 764632, WO 97/10822.

L'activité des composés a été mise en évidence à l'aide d'un test de mesure de la cicatrisation après avoir créé une lésion cutanée de 1 cm² sur le dos de souris en enlevant un fragment de peau. On a soumis des animaux diabétiques à l'étude β₃-agoniste versus placébo. Le composé a été administré par voie orale à raison de 10 mg/kg. La cicatrisation a été évaluée en mesurant quotidiennement la surface de la lésion chez les animaux traités et chez ceux ayant reçu le placébo.

Les résultats de cette étude ont montré une nette différence de cicatrisation chez les animaux traités par le composé β₃-agoniste par rapport à celle des animaux qui ont reçu du placébo, à savoir que la guérison des lésions s'achève dans un délai nettement plus bref pour les animaux traités par rapport à ceux qui ont reçu du placébo.

Grâce à cette activité cicatrisante particulière et à leur faible toxicité qui en permet l'utilisation comme médicaments, les composés β₃-agonistes peuvent bien être employés dans la prophylaxie et/ou le traitement des blessures dermiques, notamment dans la prophylaxie et/ou le traitement des plaies des membres inférieurs chez les mammifères diabétiques.

Ainsi, selon un autre de ses aspects, la présente invention concerne l'utilisation d'une quantité prophylactique et/ou efficace d'un composé β₃-agoniste pour la préparation de médicaments pour la prophylaxie et/ou le traitement des blessures, notamment chez les mammifères diabétiques.

Plus particulièrement, selon un aspect préféré, la présente invention a pour objet l'utilisation d'une quantité prophylactique et/ou efficace d'un composé choisi parmi les composés de formule (I), (II) et (III) pour la préparation de médicaments pour la prophylaxie et/ou le traitement des blessures, notamment chez les mammifères diabétiques.

La quantité de principe actif à administrer dans le traitement des blessures selon la présente invention dépend de la nature et de la gravité des affections à traiter ainsi que de la puissance du composé et du poids des malades. Généralement la dose est comprise entre 0,01 et 30 mg par kg de poids du corps, de préférence entre 0,1 et 20 mg par kg de poids du corps, notamment entre 1 et 10 mg par kg de poids du corps.

Cette dose peut être éventuellement subdivisée pendant la journée en 2, 3 ou 4 administrations. De préférence le principe actif est formulé en unités de dosage contenant de 0,1 à 400 mg, et préférablement de 0,5 à 200 mg de principe actif en combinaison avec un support pharmaceutique.

Dans le cas de traitement par voie locale, qui peut être complémentaire à une administration par voie systémique, l'unité de dosage dépend de la gravité et de l'extension des blessures ainsi que de la concentration de principe actif dans la formulation pharmaceutique.

Normalement, on utilise une crème, une pommade, un gel contenant de 0,01 à 5 %, avantageusement de 0,025 à 2,5 %, de préférence de 0,1 à 1 % de β₃-agoniste en mélange avec les excipients usuels pour l'application topique.

On peut également utiliser une lotion ou, en général, une solution ou une suspension dans laquelle le β₃-agoniste est présent à des concentrations de 0,0001 à 1%.

Dans les compositions pharmaceutiques de la présente invention pour l'administration orale, sublinguale, sous-cutanée, intramusculaire, intraveineuse, transdermique, rectale, ou locale, le principe actif peut être administré sous formes unitaires d'administration, soit tel quel par exemple sous forme lyophilisée, soit en mélange avec des supports pharmaceutiques classiques, aux animaux et aux êtres humains pour le traitement des affections susdites. Les formes unitaires d'administration appropriées comprennent les formes par voie orale telles que les comprimés éventuellement sécables, les gélules, les poudres, les granules et les solutions ou suspensions orales, les formes d'administration sublinguale et buccale, les formes d'administration sous-cutanée, intramusculaire ou intraveineuse, les formes d'administration locale et les formes d'administration rectale.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'elixir peut contenir l'ingrédient actif conjointement avec un édulcorant, acalorique de préférence, du méthylparaben et du propylparaben comme antiseptiques, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines ou des solutions stériles et injectables qui contiennent des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Pour une administration locale, on mélange le principe actif dans un excipient pour la préparation de lotions, de gels, de crèmes ou onguents ou on le dissout dans un véhicule opportun.

Dans les compositions pharmaceutiques selon la présente invention, le principe actif peut être aussi sous forme de complexe d'inclusion dans des cyclodextrines, leurs éthers ou leurs esters.

## Revendications

1. Utilisation des composés β₃-agonistes pour la préparation de médicaments cicatrisants.

2. Utilisation selon la revendication 1 pour la préparation de compositions pharmaceutiques destinées à accélerer la guérison des plaies des membres inférieurs chez les mammifères diabétiques.

3. Utilisation selon l'une des revendications 1 ou 2, **caractérisée en ce que** le β₃-agoniste est un composé de formule (I) 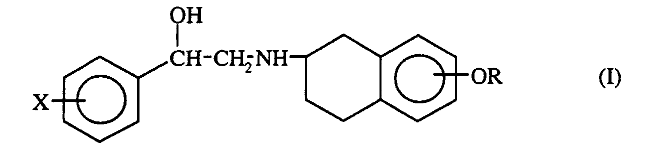 dans laquelle
X représente l'hydrogène, un halogène, un groupe trifluorométhyle ou un groupe (C₁-C₄)alkyle;
R représente l'hydrogène, un groupe méthyle, non substitué ou substitué par un groupe carbonyle ou méthoxy- ou éthoxycarbonyle
ou l'un de ses sels pharmaceutiquement acceptables.

4. Utilisation selon la revendication 3, **caractérisée en ce que** le β₃-agoniste est choisi parmi les composés ci-après :
• 2-[(7-hydroxy-1,2,3,4-tetrahydronapht-2-yl)amino]-1-phényléthanol;
• 2-[(7-hydroxy-1,2,3,4-tetrahydronapht-2-yl)amino]-1-(3-chlorophényl) éthanol;
• 2-[(7-carbéthoxyméthoxy-1,2,3,4-tetrahydronapht-2-yl)arnino]-1-(3-chlorophényl)éthanol;
• 2-[(7-carbéthoxyméthoxy-1,2,3,4-tetrahydronapht-2-yl)amino]-1-phényléthanol;
• (1R,2'RS)-2-[(7-hydroxy-1,2,3,4-tetrahydronapht-2-yl)amino]-1-phényléthanol;
• (1S,2'RS)-2-[(7-hydroxy-1,2,3,4-tetrahydronapht-2-yl)amino]-1-phényléthanol;
• (+)-(1R)-2-[(7-hydroxy-1,2,3,4-tetrahydronapht-2-yl)amino]-1-phényléthanol;
• (+)-(1S)-2-[(7-hydroxy-1,2,3,4-tetrahydronapht-2-yl)amino]-1-phényléthanol;
• (-)-(1R)-2-[(7-hydroxy-1,2,3,4-tetrahydronapht-2-yl)amino]-1-phényléthanol;
• (-)-(1S)-2-[(7-hydroxy-1,2,3,4-tetrahydronapht-2-yl)arnino]-1-phényléthanol;
• N-[(2R)-7-éthoxycarbonylméthoxy-1,2,3,4-tetrahydronapht-2-yl]-(2R)-2-(3-chlorophényl)-2-hydroxyéthanamine;
• N-[(2R)-7-méthoxycarbonylméthoxy-1,2,3,4-tetrahydronapht-2-yl]-(2R)-2-(3-chlorophényl)-2-hydroxyéthanamine;
• N-[(2S)-7-éthoxycarbonylméthoxy-1,2,3,4-tetrahydronapht-2-yl]-(2R)-2-(3-chlorophényl)-2-hydroxyéthanamine ;
et leurs sels pharmaceutiquement acceptables.

5. Utilisation selon la revendication 3, **caractérisée en ce que** le β₃-agoniste est le N-[(2R)-7-éthoxycarbonylméthoxy-1,2,3,4-tetrahydronapht-2-yl]-(2R)-2-(3-chlorophényl)-2-hydroxyéthanamine ou l'un de ses sels pharmaceutiquement acceptables.

6. Utilisation selon l'une des revendications 1 ou 2, **caractérisée en ce que** le β₃-agoniste est un composé de formule (II) 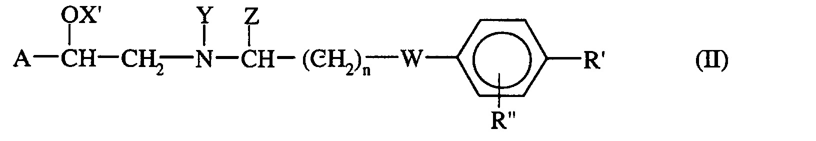 dans laquelle
n est 1, 2 ou 3;
A représente un groupe benzofuran-2-yle ou un groupe phényle non substitué ou substitué par un ou deux atomes d'halogène ou par un groupe (C₁-C₄)alkyle ou trifluorométhyle;
R' représente
- un hydrogène;
- un groupe (C₁-C₆)alkyle;
- un groupe fonctionnel choisi parmi les groupements suivants: hydroxy; (C₁-C₆)alcoxy; (C₂-C₆)alcényloxy; (C₂-C₆)alcynyloxy; (C₃-C₈)cycloalkyloxy; (C₃-C₈)cycloalkyl(C₁-C₆)alcoxy; benzyloxy; phénoxy; mercapto; (C₁-C₆)alkylethio; (C₂-C₆)alcénylthio; (C₂-C₆)alcynylthio; (C₃-C₈)cycloalkylthio; (C₃-C₈)cycloalkyl(C₁-C₆)alkylthio; benzylthio; phénylthio; ((C₁-C₆)alkyl)sulfinyle; ((C₂-C₆)alcényl)sulfinyle; ((C₂-C₆)alcynyl)sulfinyle; (C₃-C₈)cycloalkylsulfinyle; ((C₃-C₈)cycloalkyl(C₁-C₆)alkyl)sulfinyle; benzylsulfinyle; phénylsulfinyle; ((C₁-C₆)alkyl)sulfonyle; ((C₂-C₆)alcényl)sulfonyle; ((C₂-C₆)alcynyl)sulfonyle; (C₃-C₈)cycloalkylsulfonyle; ((C₃-C₈)cycloalkyl(C₁-C₆)alkyl)sulfonyle; benzylsulfonyle; phénylsulfonyle; cyano; nitro; amino non substitué ou substitué par un ou deux radicaux, identiques ou différents choisis parmi (C₁-C₆)alkyle, (C₂-C₆)alcényle, (C₂-C₆)alcynyle, (C₃-C₈)cycloalkyle, (C₃-C₈)cycloalkyl(C₁-C₆)alkyle, benzyle, phényle; carboxy; carbalcoxy dont le groupe alkyle est en C₁-C₆; ((C₂-C₆)alcényloxy)carbonyle; ((C₂-C₆)alcynyloxy)carbonyle; (C₃-C₈)cycloalkyloxycarbonyle; ((C₃-C₈)cycloalkyl(C₁-C₆)alcoxy)carbonyle; benzyloxycarbonyle; phénoxycarbonyle; carbamoyle non substitué ou substitué sur le groupe amino par un ou deux radicaux, identiques ou différents, choisis parmi les groupes (C₁-C₆)alkyle, (C₂-C₆)alcényle, (C₂-C₆)alcynyle, (C₃-C₈)cycloalkyle, (C₃-C₈)cycloalkyl(C₁-C₆)alkyle, benzyle et phényle;
- un groupe R''' choisi parmi les groupes (C₁-C₆)alkyle substitué par un groupe fonctionnel, (C₂-C₆)alcényle substitué par un groupe fonctionnel; (C₂-C₆)alcynyle substitué par un groupe fonctionnel; phényl (C₁-C₆)alkyle substitué sur le groupe phényle par un (C₁-C₆)alkyle ou par un groupe fonctionnel; phényl(C₂-C₆)alcényle substitué sur le groupe phényle par un (C₁-C₆)alkyle ou par un groupe fonctionnel; phényl(C₂-C₆)alcynyle substitué sur le groupe phényle par un (C₁-C₆)alkyle ou par un groupe fonctionnel; benzyle substitué par un (C₁-C₆)alkyle ou par un groupe fonctionnel; phényle, non substitué ou substitué par un (C₁-C₆)alkyle ou par un groupe fonctionnel; le groupe fonctionnel étant tel que défini ci-dessus pour R';
- un groupe O-R''', S-R"', SO-R"', SO₂R''' dans lesquels R''' est tel que défini ci-dessus pour R';
- un groupe NR'''R°, où R''' est tel que défini ci-dessus et R° représente l'hydrogène ou est tel que défini ci-dessus pour R''', ou bien R''' et R° forment, avec l'azote auquel ils sont liés, un groupe choisi parmi les groupes pyrrolidino, pipéridino et morpholino;
- un groupe COOR''' ou un groupe CO-SR''' dans lesquels R'''est tel que défini ci-dessus;
- un groupe CONR'''R° où R''' est tel que défini ci-dessus et R° représente l'hydrogène ou est tel que défini ci-dessus pour R"', ou bien R''' et R° forment, avec l'azote auquel ils sont liés, un groupe choisi parmi les groupes pyrrolidino, pipéridino et morpholino;
- un groupe SO₂NR'''R° où R''' est tel que défini ci-dessus et R° représente l'hydrogène ou est tel que défini ci-dessus pour R"', ou bien R''' et R° forment, avec l'azote auquel ils sont liés, un groupe choisi parmi les groupes pyrrolidino, pipéridino et morpholino;
R" représente
- un hydrogène;
- un halogène;
- un groupe (C₁-C₆)alkyle;
- un groupe fonctionnel tel que défini ci-dessus pour R';
- un groupe OR''', R''' étant tel que défini ci-dessus;
- un groupe COOR''', R''' étant tel que défini ci-dessus;
- un groupe CONR'''R° où R''' est tel que défini ci-dessus et R° représente l'hydrogène ou est tel que défini ci-dessus pour R"', ou bien R''' et R° forment, avec l'azote auquel ils sont liés, un groupe choisi parmi les groupes pyrrolidino, pipéridino et morpholino;
W représente une liaison directe ou un atome d'oxygène;
X' représente l'hydrogène, un (C₁-C₆)alkyle ou un (C₁-C₆)alkylcarbonyle;
Y représente l'hydrogène ou un groupe A'-CH(OH)-CH₂-, A' étant identique à A, mais autre que benzofuran-2-yle; ou bien
X' et Y, pris ensemble, forment un groupe méthylène, éventuellement substitué par un groupe carbalcoxy dont le groupe alkyle est en C₁-C₆; un groupe éthylène, éventuellement substitué par un groupe oxo; ou un groupe 1,3-propylène;
Z représente l'hydrogène ou un (C₁-C₆)alkyle;
ou l'un de ses sels pharmaceutiquement acceptables.

7. Utilisation selon l'une des revendications 1 ou 2, **caractérisée en ce que** le β₃-agoniste est un composé de formule (III) 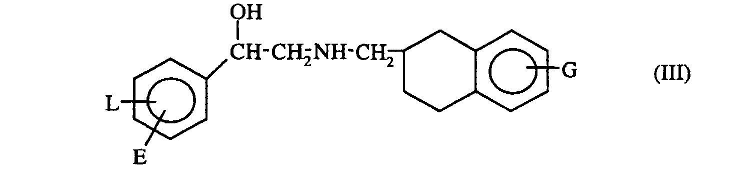 dans laquelle
E représente l'hydrogène, un groupe (C₁-C₄)alkyle, un groupe (C₁-C₄)alcoxy, un groupe phényle, un groupe nitro, un atome d'halogène ou un groupe trifluorométhyle,
L représente l'hydrogène, un groupe (C₁-C₄)alkyle, un groupe (C₁-C₄)alcoxy, un groupe phényle, un groupe nitro, un atome d'halogène ou
E et L , ensemble, représentent un groupe -CH=CH-CH=CH- ou -CH₂-CH₂-CH₂-CH₂-, et
G représente l'hydrogène, un atome de chlore, un groupe hydroxy ou un groupe OG' où G' représente un groupe (C₁-C₄)alkyle non substitué ou substitué par un groupe hydroxy, (C₁-C₄)alcoxy, (C₁-C₄)alcoxycarbonyle, carboxy ou (C₃-C₇)cycloalkyle; un groupe (C₃-C₇)cycloalkyle; un groupe (C₂-C₄)alcanoyle,
ou un de ses sels pharmaceutiquement acceptables.

8. Utilisation selon la revendication 7, **caractérisée en ce que** le β₃-agoniste est choisi parmi la N-[(6-hydroxy-1,2,3,4-tétrahydronaphtalen-(2R)-2-yl)méthyl]-(2R)-2-hydroxy-2-(3-chlorophényl)éthanamine, la N-[(7-méthoxy-1,2,3,4-tétrahydronaphtalen-(2R)-2-yl)méthyl]-(2R)-2-hydroxy-2-(3-chlorophényl)éthanamine, et leurs sels pharmaceutiquement acceptables.

9. Composition pharmaceutique pour administration locale, **caractérisée en ce qu'**elle contient de 0,01 à 5 % d'un β₃-agoniste comme principe actif.

10. Composition pharmaceutique selon la revendication 9, **caractérisée en ce qu'**elle contient de 0,025 à 2,5 % de principe actif.

11. Composition pharmaceutique selon la revendication 10, **caractérisée en ce qu'**elle contient de 0,1 à 1 % de principe actif.

## Patentansprüche

1. Verwendung von β₃-Agonisten zur Herstellung von Wundheilmitteln.

2. Verwendung nach Anspruch 1 zur Herstellung von pharmazeutischen Zusammensetzungen, die dazu vorgesehen sind, die Heilung von Wunden der unteren Gliedmaßen bei diabetischen Säugern zu beschleunigen.

3. Verwendung nach einem der Ansprüche 1 oder 2, **dadurch gekennzeichnet, dass** es sich bei dem β₃-Agonisten um eine Verbindung der Formel (I): 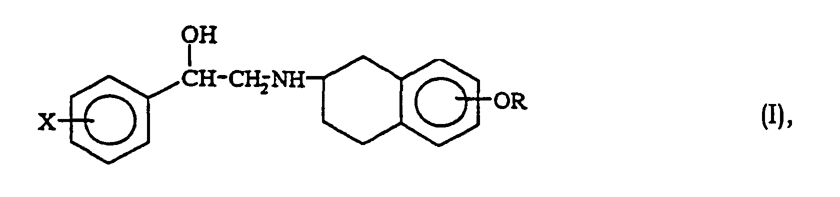 worin bedeuten:
X Wasserstoff, Halogen, Trifluormethyl oder C₁₋₄-Alkyl; und
R Wasserstoff oder eine Methylgruppe, die unsubstituiert oder mit einer Carboxygruppe oder Methoxy- oder Ethoxycarbonylgruppe substituiert ist;
oder ein pharmazeutisch akzeptables Salz dieser Verbindungen handelt.

4. Verwendung nach Anspruch 3, **dadurch gekennzeichnet, dass** der β₃-Agonist unter den folgenden Verbindungen ausgewählt ist:
· 2-[(7-Hydroxy-1,2,3,4-tetrahydronapht-2-yl)amino]-1-phenylethanol;
· 2-[(7-Hydroxy-1,2,3,4-tetrahydronapht-2-yl)amino]-1-(3-chlorphenyl)ethanol;
· 2-[(7-Carbethoxymethoxy-1,2,3,4-tetrahydronapht-2-yl)amino]-1-(3-chlorphenyl)ethanol;
· 2-[(7-Carbethoxymethoxy-1,2,3,4-tetrahydronapht-2-yl)amino]-1-phenylethanol;
· (1R,2'RS)-2-[(7-Hydroxy-1,2,3,4-tetrahydronapht-2-yl)amino]-1-phenylethanol;
· (1S,2'RS)-2-[(7-Hydroxy-1,2,3,4-tetrahydronapht-2-yl)amino]-1-phenylethanol;
· (+)-(1R)-2-[(7-Hydroxy-1,2,3,4-tetrahydronapht-2-yl)amino]-1-phenylethanol;
· (+)-(1S)-2-[(7-Hydroxy-1,2,3,4-tetrahydronapht-2-yl)amino]-1-phenylethanol;
· (-)-(1R)-2-[(7-Hydroxy-1,2,3,4-tetrahydronapht-2-yl)amino]-1-phenylethanol;
· (-)-(1S)-2-[(7-Hydroxy-1,2,3,4-tetrahydronapht-2-yl)amino]-1-phenylethanol;
· N-[(2R)-7-Ethoxycarbonylmethoxy-1,2,3,4-tetrahydronapht-2-yl]-(2R)-2-(3-chlorphenyl)-2-hydroxyethanamin;
· N-[(2R)-7-Methoxycarbonylmethoxy-1,2,3,4-tetrahydronapht-2-yl]-(2R)-2-(3-chlorphenyl)-2-hydroxyethanamin;
· N-[(2S)-7-Ethoxycarbonylmethoxy-1,2,3,4-tetrahydronapht-2-yl]-(2R)-2-(3-chlorphenyl)-2-hydroxyethanamin;
und deren pharmazeutisch akzeptablen Salzen.

5. Verwendung nach Anspruch 3, **dadurch gekennzeichnet, dass** der β₃-Agonist das N-[(2R)-7-Ethoxycarbonylmethoxy-1,2,3,4-tetrahydronapht-2-yl]-(2R)-2-(3-chlorphenyl)-2-hydroxyethanamin oder ein pharmazeutisch akzeptables Salz dieser Verbindung ist.

6. Verwendung nach einem der Ansprüche 1 oder 2, **dadurch gekennzeichnet, dass** der β₃-Agonist eine Verbindung der folgenden Formel (II) ist: 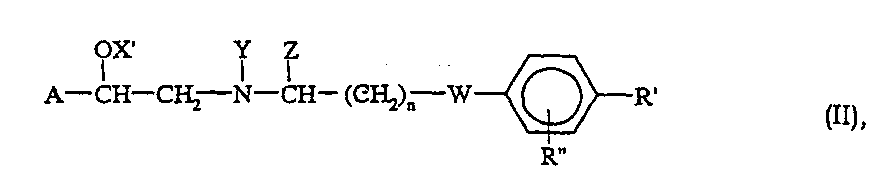 worin bedeuten:
n 1, 2 oder 3;
A Benzofuran-2-yl oder eine Phenylgruppe, die unsubstituiert vorliegt oder mit einem oder zwei Halogenatomen oder einer C₁₋₄-Alkylgruppe oder Trifluormethylgruppe substituiert ist;
R'
- Wasserstoff;
- C₁₋₆-Alkyl;
- eine funktionelle Gruppe, die unter den folgenden Gruppen ausgewählt ist: Hydroxy; C₁₋₆-Alkoxy; C₂₋₆-Alkenyloxy; C₂₋₆-Alkinyloxy; C₃₋₈-Cycloalkyloxy; C₃₋₈-Cycloalkyl-C₁₋₆-alkoxy; Benzyloxy; Phenoxy; Mercapto; C₁₋₆-Alkylthio; C₂₋₆-Alkenylthio; C₂₋₆-Alkinylthio; C₃₋₈-Cycloalkylthio; C₃₋₈-Cycloalkyl-C₁₋₆-alkylthio; Benzylthio; Phenylthio; (C₁₋₆-Alkyl)sulfinyl; (C₂₋₆-Alkenyl)sulfinyl; (C₂₋₆-Alkinyl)sulfinyl; C₃₋₈-Cycloalkylsulfinyl; (C₃₋₈-Cycloalkyl-C₁₋₆-alkyl)sulfinyl; Benzylsulfinyl; Phenylsulfinyl; (C₁₋₆-Alkyl)sulfonyl; (C₂₋₆-Alkenyl)sulfonyl; (C₂₋₆-Alkinyl)-sulfonyl; C₃₋₈-Cycloalkylsulfonyl; (C₃₋₈-Cycloalkyl-C₁₋₆-alkyl)sulfonyl; Benzylsulfonyl; Phenylsulfonyl; Cyano; Nitro; eine Aminogruppe, die unsubstituiert vorliegt oder mit einer oder zwei Gruppen substituiert ist, die gleich oder verschieden sind und unter C₁₋₆-Alkyl, C₂₋₆-Alkenyl, C₂₋₆-Alkinyl, C₃₋₈-Cycloalkyl, C₃₋₈-Cycloalkyl-C₁₋₆-alkyl, Benzyl oder Phenyl ausgewählt sind; Carboxy, Carbalkoxy, dessen Alkylgruppe 1 bis 6 Kohlenstoffatome enthält; (C₂₋₆-Alkenyloxy)carbonyl; (C₂₋₆-Alkinyloxy)carbonyl; (C₃₋₈-Cycloalkyloxy)carbonyl; (C₃₋₈-Cycloalkyl-C₁₋₆-alkoxy)-carbonyl; Benzyloxycarbonyl; Phenoxycarbonyl; Carbamoyl, das unsubstituiert vorliegt oder an der Aminogruppe mit einer oder zwei Gruppen substituiert ist, die gleich oder verschieden sind und unter C₁₋₆-Alkyl, C₂₋₆-Alkenyl, C₂₋₆-Alkinyl, C₃₋₈-Cycloalkyl, C₃₋₈-Cycloalkyl-C₁₋₆-alkyl, Benzyl und Phenyl ausgewählt sind;
- eine Gruppe R'", die unter den mit einer funktionellen Gruppe substituierten C₁₋₆-Alkylgruppen; den mit einer funktionellen Gruppe substituierten C₂₋₆-Alkenylgruppen; den mit einer funktionellen Gruppe substituierten C₂₋₆-Alkinylgruppen; den an der Phenylgruppe mit einer C₁₋₆-Alkylgruppe oder einer funktionellen Gruppe substituierten Phenyl-C₁₋₆-alkylgruppen; den an der Phenylgruppe mit einer C₁₋₆-Alkylgruppe oder einer funktionellen Gruppe substituierten Phenyl-C₂₋₆-alkenylgruppen; den an der Phenylgruppe mit einer C₁₋₆-Alkylgruppe oder einer funktionellen Gruppe substituierten Phenyl-C₂₋₆-alkinylgruppen; den mit einer C₁₋₆-Alkylgruppe oder einer funktionellen Gruppe substituierten Benzylgruppen; und einer unsubstituierten Phenylgruppe oder mit einer C₁₋₆-Alkylgruppe oder einer funktionellen Gruppe substituierten Phenylgruppen ausgewählt ist; wobei die funktionelle Gruppe die oben für R' angegebenen Bedeutungen aufweist;
- eine Gruppe O-R'", S-R'", SO-R'", SO₂R'", wobei R'" die oben für R' angegebenen Bedeutungen aufweist;
- eine Gruppe NR'"R⁰, wobei R'" die oben angegebenen Bedeutungen aufweist und R⁰ Wasserstoff bedeutet oder die für R'" angegebenen Bedeutungen aufweist, oder R'" und R⁰ bilden gemeinsam mit dem Stickstoffatom, an das sie gebunden sind, eine Gruppe, die unter den Gruppen Pyrrolidino, Piperidino und Morpholino ausgewählt ist;
- eine Gruppe COOR'" oder eine Gruppe CO-SR"', wobei R'" die oben angegebenen Bedeutungen aufweist;
- eine Gruppe CONR'"R⁰, wobei R'" die oben angegebenen Bedeutungen aufweist und R⁰ Wasserstoff bedeutet oder die oben für R'" angegebenen Bedeutungen aufweist, oder R'" und R⁰ bilden gemeinsam mit dem Stickstoffatom, an das sie gebunden sind, eine Gruppe, die unter den Gruppen Pyrrolidino, Piperidino und Morpholino ausgewählt ist;
- eine Gruppe SO₂NR'"R⁰, wobei R'" die oben angegebenen Bedeutungen aufweist und R⁰ Wasserstoff bedeutet oder die oben für R'" angegebenen Bedeutungen aufweist, oder R'" und R⁰ bilden gemeinsam mit dem Stickstoffatom, an das sie gebunden sind, eine Gruppe, die unter den Gruppen Pyrrolidino, Piperidino und Morpholino ausgewählt ist;
R"
- Wasserstoff;
- Halogen;
- C₁₋₆-Alkyl;
- eine funktionelle Gruppe, wie sie oben für R' definiert wurde;
- eine Gruppe OR'", wobei R'" die oben angegebenen Bedeutungen aufweist;
- eine Gruppe COOR'", wobei R'" die oben angegebenen Bedeutungen aufweist;
- eine Gruppe CONR'"R⁰, wobei R'" die oben angegebenen Bedeutungen aufweist und R⁰ Wasserstoff bedeutet oder die oben für R'" angegebenen Bedeutungen aufweist, oder R'" und R⁰ bilden gemeinsam mit dem Stickstoffatom, an das sie gebunden sind, eine Gruppe, die unter den Gruppen Pyrrolidino, Piperidino und Morpholino ausgewählt ist;
W eine direkte Bindung oder ein Sauerstoffatom;
X' Wasserstoff, C₁₋₆-Alkyl oder C₁₋₆-Alkylcarbonyl;
Y Wasserstoff oder eine Gruppe A'-CH(OH)-CH₂-, wobei A' mit A identisch, jedoch von Benzofuran-2-yl verschieden ist; oder
X' und Y bilden gemeinsam eine Methylengruppe, die gegebenenfalls mit einer Carbalkoxygruppe substituiert ist, deren Alkylgruppe 1 bis 6 Kohlenstoffatome aufweist; eine Ethylengruppe, die gegebenenfalls mit einer Oxogruppe substituiert ist; oder eine 1,3-Propylengruppe; und
Z Wasserstoff oder C₁₋₆-Alkyl;
oder ein pharmazeutisch akzeptables Salz dieser Verbindungen.

7. Verwendung nach einem der Ansprüche 1 oder 2, **dadurch gekennzeichnet, dass** der β₃-Agonist eine Verbindung der folgenden Formel (III) ist: 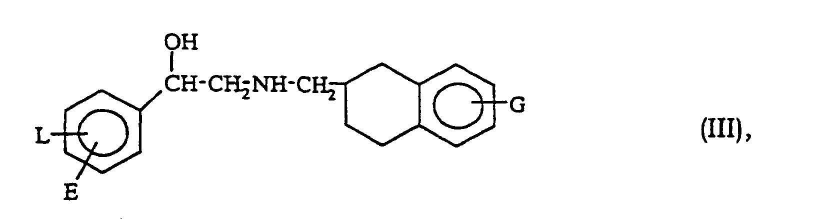 worin bedeuten:
E Wasserstoff, C₁₋₄-Alkyl, C₁₋₄-Alkoxy, Phenyl, Nitro, Halogen oder Trifluormethyl,
L Wasserstoff, C₁₋₄-Alkyl, C₁₋₄-Alkoxy, Phenyl, Nitro oder Halogen, oder
E und L bedeuten gemeinsam eine Gruppe -CH=CH-CH=CH- oder -CH₂-CH₂-CH₂-CH₂-, und
G Wasserstoff, Chlor, Hydroxy oder eine Gruppe OG', wobei G' eine unsubstituierte C₁₋₄-Alkylgruppe oder eine C₁₋₄-Alkylgruppe ist, die mit einer Hydroxygruppe, C₁₋₄-Alkoxy, C₁₋₄-Alkoxycarbonyl, Carboxy oder C₃₋₇-Cycloalkyl substituiert ist; C₃₋₇-Cycloalkyl; C₂₋₄-Alkanoyl,
oder ein pharmazeutisch akzeptables Salz dieser Verbindungen.

8. Verwendung nach Anspruch 7, **dadurch gekennzeichnet, dass** der β₃-Agonist unter N-[(6-Hydroxy-1,2,3,4-tetrahydronaphthalin-(2R)-2-yl)methyl]-(2R)-2-hydroxy-2-(3-chlorphenyl)-ethanamin, N-[(7-Methoxy-1,2,3,4-tetrahydronaphthalin-(2R)-2-yl)methyl]-(2R)-2-hydroxy-2-(3-chlorphenyl)ethanamin und deren pharmazeutisch akzeptablen Salzen ausgewählt ist.

9. Pharmazeutische Zusammensetzung zur lokalen Verabreichung, **dadurch gekennzeichnet, dass** sie 0,01 bis 5 % eines β₃-Agonisten als Wirkstoff enthält.

10. Pharmazeutische Zusammensetzung nach Anspruch 9, **dadurch gekennzeichnet, dass** sie 0,025 bis 2,5 % Wirkstoff enthält.

11. Pharmazeutische Zusammensetzung nach Anspruch 10, **dadurch gekennzeichnet, dass** sie 0,1 bis 1 % Wirkstoff enthält.

## Claims

1. Use of β₃-agonist compounds for the preparation of healing drugs.

2. Use according to claim 1 for the preparation of pharmaceutical compositions intended for accelerating the healing of wounds in the lower limbs of diabetic mammals.

3. Use according to any one of claims 1 or 2, **characterized in that** the β₃-agonist is a compound of formula (I): 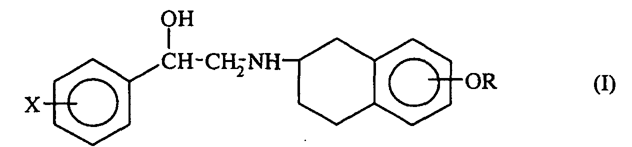 in which:
X is hydrogen, a halogen, a trifluoromethyl group or a (C₁-C₄)alkyl group; and
R is hydrogen or a methyl group which is unsubstituted or substituted by a carboxyl or a methoxy- or ethoxycarbonyl group,
or one of its pharmaceutically acceptable salts.

4. Use according to claim 3, **characterized in that** the β₃-agonist is selected from the following compounds :
• 2-[(7-hydroxy-1,2,3,4-tetrahydronaphth-2-yl)amino]-1-phenylethanol;
• 2-[(7-hydroxy-1,2,3,4-tetrahydronaphth-2-yl)amino]-1-(3-chlorophenyl)ethanol;
• 2-[(7-carbethoxymethoxy-1,2,3,4-tetrahydronaphth-2-yl)amino]-1-(3-chlorophenyl)ethanol;
• 2-[(7-carbethoxymethoxy-1,2,3,4-tetrahydronaphth-2-yl)amino]-1-pheny)ethanol;
• (1R,2'RS)-2-[(7-hydroxy-1,2,3,4-tetrahydronaphth-2-yl)amino]-1-phenylethanol;
• (1S,2'RS)-2-[(7-hydroxy-1,2,3,4-tetrahydronaphth-2-yl)amino]-1-phenylethanol;
• (+)-(1R)-2-[(7-hydroxy-1,2,3,4-tetrahydronaphth-2-yl)amino]-1-phenylethanol;
• (+)-(1S)-2-[(7-hydroxy-1,2,3,4-tetrahydronaphth-2-yl)amino]-1-phenylethanol;
• (-)-(1R)-2-[(7-hydroxy-1,2,3,4-tetrahydronaphth-2-yl)amino]-1-phenylethanol;
• (-)-(1S)-2-[(7-hydroxy-1,2,3,4-tetrahydronaphth-2-yl)amino]-1-phenylethanol;
• N-[(2R)-7-ethoxycarbonylmethoxy-1,2,3,4-tetrahydronaphth-2-yl]-(2R)-2-(3-chlorophenyl)-2-hydroxyethanamine;
• N-[(2R)-7-methoxycarbonylmethoxy-1,2,3,4-tetrahydronaphth-2-yl]-(2R)-2-(3-chlorophenyl)-2-hydroxyethanamine;
• N-[(2S)-7-ethoxycarbonylmethoxy-1,2,3,4-tetrahydronaphth-2-yl]-(2R)-2-(3-chlorophenyl)-2-hydroxyethanamine ;
and their pharmaceutically acceptable salts.

5. Use according to claim 3, **characterized in that** the β₃-agonist is N-[(2R)-7-ethoxycarbonylmethoxy-1,2,3,4-tetrahydronaphth-2-yl]-(2R)-2-(3-chlorophenyl)-2-hydroxyethanamine or one of its pharmaceutically acceptable salts.

6. Use according to any one of claims 1 or 2, **characterized in that** the β₃-agonist is a compound of formula (II): 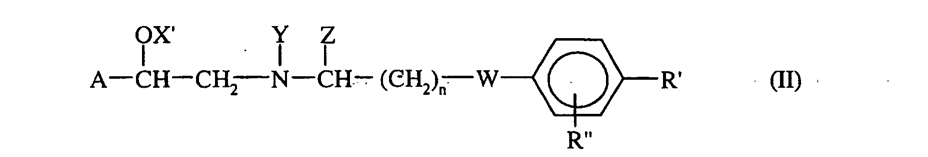 in which:
n is 1, 2 or 3;
A is a benzofuran-2-yl group or a phenyl group which is unsubstituted or substituted by one or two halogen atoms or by a (C₁-C₄)alkyl or trifluoromethyl group;
R' is:
- a hydrogen;
- a (C₁-C₆)alkyl group;
- a functional group selected from the following groups: hydroxyl; (C₁-C₆)alkoxy; (C₂-C₆)alkenyloxy; (C₂-C₆)alkynyloxy; (C₃-C₈)cycloalkoxy; (C₃-C₈)cycloalkyl(C₁-C₆)alkoxy; benzyloxy; phenoxy; mercapto; (C₁-C₆)alkylthio; (C₂-C₆)alkenylthio; (C₂-C₆)alkynylthio; (C₃-C₈)cycloalkylthio; (C₃-C₈)cycloalkyl(C₁-C₆)alkylthio; benzylthio; phenylthio; ((C₁-C₆)alkyl)sulfinyl; ((C₂-C₆)alkenyl)sulfinyl; ((C₂-C₆)alkynyl)sulfinyl; (C₃-C₈)cycloalkylsulfinyl; ((C₃-C₈)cycloalkyl(C₁-C₆)alkyl)sulfinyl; benzylsulfinyl; phenylsulfinyl; ((C₁-C₆)alkyl)sulfonyl; ((C₂-C₆)alkenyl)sulfonyl; ((C₂-C₆)alkynyl)sulfonyl; (C₃-C₈)cycloalkylsulfonyl; ((C₃-C₈)cycloalkyl(C₁-C₆)alkyl)sulfonyl; benzylsulfonyl; phenylsulfonyl; cyano; nitro; amino which is unsubstituted or substituted by one or two identical or different radicals selected from (C₁-C₆)alkyl, (C₂-C₆)alkenyl, (C₂-C₆)alkynyl, (C₃-C₈)cycloalkyl, (C₃-C₈)cycloalkyl(C₁-C₆)alkyl, benzyl, phenyl; carboxyl; carbalkoxy in which the alkyl group is C₁-C₆; ((C₂-C₆)alkenyloxy)carbonyl; ((C₂-C₆)alkynyloxy)carbonyl; (C₃-C₈)cycloalkoxycarbonyl; ((C₃-C₈)cycloalkyl(C₁-C₆)alkoxy)carbonyl; benzyloxycarbonyl; phenoxycarbonyl; carbamoyl which is unsubstituted or substituted on the amino group by one or two identical or different radicals selected from (C₁-C₆)alkyl, (C₂-C₆)alkenyl, (C₂-C₆)alkynyl, (C₃-C₈)cycloalkyl, (C₃-C₈)cycloalkyl(C₁-C₆)alkyl, benzyl and phenyl groups;
- a group R''' selected from the groups: (C₁-C₆)alkyl substituted by a functional group; (C₂-C₆)alkenyl substituted by a functional group; (C₂-C₆)alkynyl substituted by a functional group; phenyl(C₁-C₆)alkyl substituted on the phenyl group by a (C₁-C₆)alkyl or by a functional group; phenyl(C₂-C₆)alkenyl substituted on the phenyl group by a (C₁-C₆)alkyl or by a functional group; phenyl(C₂-C₆)alkynyl substituted on the phenyl group by a (C₁-C₆)alkyl or by a functional group; benzyl substituted by a (C₁-C₆)alkyl or by a functional group; and phenyl which is unsubstituted or substituted by a (C₁-C₆)alkyl or by a functional group, the functional group being as defined above for R';
- a group O-R''', S-R''', SO-R''' or SO₂R''', in which R''' is as defined above;
- a group NR'''R°, in which R''' is as defined above and R° is hydrogen or is as defined above for R''', or R''' and R° form, together with the nitrogen to which they are bonded, a group selected from pyrrolidino, piperidino and morpholino groups;
- a group COOR''' or a group CO-SR"', in which R''' is as defined above;
- a group CONR'''R°, in which R"' is as defined above and R° is hydrogen or is as defined above for R''', or R''' and R° form, together with the nitrogen to which they are bonded, a group selected from pyrrolidino, piperidino and morpholino groups; or
- a group SO₂NR'''R°, in which R''' is as defined above and R° is hydrogen or is as defined above for R''', or R''' and R° form, together with the nitrogen to which they are bonded, a group selected from pyrrolidino, piperidino and morpholino groups;
R" is:
- a hydrogen;
- a halogen;
- a (C₁-C₆)alkyl group;
- a functional group as defined above for R';
- a group OR''', R''' being as defined above;
- a group COOR"', R''' being as defined above; or
- a group CONR'''R°, in which R''' is as defined above and R° is hydrogen or is as defined above for R''', or R''' and R° form, together with the nitrogen to which they are bonded, a group selected from pyrrolidino, piperidino and morpholino groups;
W is a direct bond or an oxygen atom;
X' is hydrogen, a (C₁-C₆)alkyl or a (C₁-C₆)alkylcarbonyl;
Y is hydrogen or a group A'-CH(OH)-CH₂-, A' being identical to A but other than benzofuran-2-yl; or
X' and Y, taken together, form a methylene group optionally substituted by a carbalkoxy group in which the alkyl group is C₁-C₆; an ethylene group optionally substituted by an oxo group; or a 1,3-propylene group;
Z is hydrogen or a (C₁-C₆)alkyl,
or one of its pharmaceutically acceptable salts.

7. Use according to any one of claims 1 or 2, **characterized in that** the β₃-agonist is a compound of formula (III): 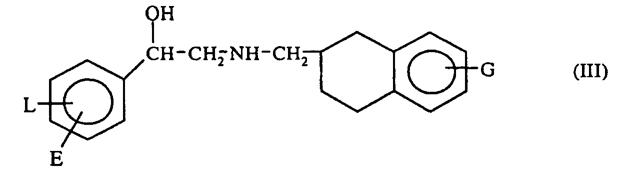 in which:
E is hydrogen, a (C₁-C₄)alkyl group, a (C₁-C₄)alkoxy group, a phenyl group, a nitro group, a halogen atom or a trifluoromethyl group;
L is hydrogen, a (C₁-C₄)alkyl group, a (C₁-C₄)alkoxy group, a phenyl group, a nitro group or a halogen atom; or
E and L together are a group -CH=CH-CH=CH- or -CH₂-CH₂-CH₂-CH₂-; and
G is hydrogen, a chlorine atom, a hydroxyl group or a group OG', in which G' is a (C₁-C₄)alkyl group which is unsubstituted or substituted by a hydroxyl, (C₁-C₄)alkoxy, (C₁-C₄)alkoxycarbonyl, carboxyl or (C₃-C₇)cycloalkyl group; a (C₃-C₇)cycloalkyl group; or a (C₂-C₄)alkanoyl group,
or one of its pharmaceutically acceptable salts.

8. Use according to claim 7, **characterized in that** the β₃-agonist is selected from N-[(6-hydroxy-1,2,3,4-tetrahydronaphthalen-(2R)-2-yl)methyl]-(2R)-2-hydroxy-2-(3-chlorophenyl)ethanamine, N-[(7-methoxy-1,2,3,4-tetrahydronaphthalen-(2R)-2-yl)methyl]-(2R)-2-hydroxy-2-(3-chlorophenyl)ethanamine and their pharmaceutically acceptable salts.

9. A pharmaceutical composition for topic administration, **characterized in that** it contains from 0.01 to 5% of a β₃-agonist as active principle.

10. A pharmaceutical composition according to claim 9, **characterized in that** it contains from 0.025 to 2.5% of active principle.

11. A pharmaceutical composition according to claim 10, **characterized in that** it contains from 0.1 to 1 % of active principle.
